# EUROPÄISCHE PATENTSCHRIFT

(11) **EP 1 103 227 B1**
(45) Veröffentlichungstag und Bekanntmachung des Hinweises auf die Patenterteilung: **11.10.2006**
(21) Anmeldenummer: 00810999.3
(22) Anmeldetag: 27.10.2000
(51) Int. Cl.: A61B 17/88

(54) **Operationswerkzeug**
Medical operation tool
Outil médical d'opération

(30) Priorität: 26.11.1999 EP 99811092
(43) Veröffentlichungstag der Anmeldung: 30.05.2001
(73) Patentinhaber: Zimmer GmbH, 8404 Winterthur (CH)
(72) Erfinder: Casutt, Simon, 9200 Gossau (CH)
(74) Vertreter: Manitz, Finsterwald & Partner GbR

(56) Entgegenhaltungen:
- FR-A- 2 702 172
- GB-A- 943 610
- US-A- 1 630 239
- US-A- 2 288 584

## Beschreibung

Die Erfindung betrifft ein Operationswerkzeug für den Einsatz im Operationssaal gemäß dem Oberbegriff des Anspruchs 1 sowie einen Operationswerkzeugsatz gemäß dem Oberbegriff des Anspruchs 7. Solche Operationswerkzeuge weisen grundsätzlich ein Griffteil auf, welches der operierende Arzt greift und mit welchem er das Werkzeug bedienen kann, sowie ein Werkzeugteil, mit welchem die Bearbeitung erfolgt. Griffteil und Werkzeugteil sind fest miteinander verbunden.

Des Weiteren sind z.B. aus US 2,288,584, FR 2 702 172 A1 und GB 943,610 A Werkzeuge bekannt, bei denen zwischen einem Griff und einem Werkzeug eine lösbare Verbindung besteht, insbesondere eine Schraubverbindung mittels einer ein Innengewinde aufweisenden Kopplungshülse, die mit einem Augenwinkel einer Griffverlängerung zusammenwirkt (US 2.288.584).

Operationswerkzeuge für den Einsatz im Operationssaal gibt es in unzähligen Ausführungen. Die je nach Einsatzgebiet höchst unterschiedlichen Werkzeuge müssen nach einer Operation einfach und zuverlässig reinigbar und sterilisierbar sein, sofern es sich um wiederverwendbare Werkzeuge handelt.

Bereits existierende Operationswerkzeuge haben Griffteile aus einem Hartkunststoff, die je nach Einsatzgebiet sehr unterschiedlich ausgebildet sind. Existierende Griffteile lassen sich in verschiedene Typen unterteilen, von denen einige im folgenden beispielhaft genannt werden. Ein Beispiel für einen solchen Typ von Griffteil ist ein Schraubendrehergriff, also ein längliches, profiliertes Griffteil, mit welchem insbesondere Druckkräfte und Drehmomente ausgeübt werden können. Ein weiteres Beispiel für ein solches Griffteil ist der sogenannte T-Griff, mit welchem insbesondere Zugkräfte und Drehmomente ausgeübt werden können. Ein anderes Beispiel für ein solches Griffteil ist der Knollen-Griff, mit welchem insbesondere Druckkräfte und seitliche gerichtete Hebelkräfte ausgeübt werden können. Wieder ein anderes Beispiel für ein solches Griffteil ist der Tastgriff, ein im wesentlichen unprofilierter länglicher Griff, welche insbesondere für Einsätze geeignet ist, mit welchem bestimmte Gegebenheiten ertastet werden. Darüberhinaus gibt es noch etliche andere Typen von Griffen, die hier nicht alle einzeln aufgezählt werden können.

Bei bereits existierenden Operationswerkzeugen ist es so, dass die Werkzeugteile und die Griffteile nicht nur fest, sondern darüberhinaus auch unlösbar miteinander verbunden sind. Häufig sind die typischerweise aus Edelstahl oder einem anderen nicht-rostenden Material bestehenden Werkzeugteile in das typischerweise aus Kunststoff bestehende Griffteil eingegossen bzw. von dem Kunststoff des Griffteils umspritzt worden. Derartige Operationswerkzeuge lassen sich auf einfache und zuverlässige Weise reinigen (z.B. mittels Ultraschallbädern) und auch sterilisieren (z.B. durch Dampfsterilisation).

Die unzähligen Ausführungen der verschiedenen Arten von Operationswerkzeugen, die dann regelmässig auch noch in etlichen verschiedenen Grössen vorhanden sein müssen, erfordern einen hohen Lageraufwand, weil jedes Werkzeugteil mit seinem dazugehörigen Griffteil in den verschiedenen Grössen gelagert werden muss. Häufig ist es sogar noch so, dass ein Werkzeugteil mit verschiedenen Typen von Griffteilen vorhanden sein muss, weil der operierende Arzt je nach Einsatzgebiet zwar dasselbe Werkzeugteil benötigt, jedoch ein unterschiedliches Griffteil.

Bisher muss für jede gewünschte Kombination von Griffteil und Werkzeugteil und für jede gewünschte Grösse jeweils ein separates Operationswerkzeug vorhanden sein. Dies erhöht den Lageraufwand noch weiter, erst recht, wenn man berücksichtigt, dass pro Kombination von Griffteil und Werkzeugteil in der Regel mehrere Exemplare vorrätig sein müssen, einmal ganz abgesehen von den hohen Anschaffungskosten, die natürlich mit einer entsprechend grossen Anzahl von Operationswerkzeugen einhergehen.

Hier will die Erfindung Abhilfe schaffen. Demzufolge ist es eine Aufgabe der Erfindung, ein Operationswerkzeug vorzuschlagen, mit welchem es möglich ist, den Lageraufwand für Operationswerkzeuge erheblich zu reduzieren, ohne aber die Vielfalt und die Verfügbarkeit der verschiedenen Operationswerkzeuge zu verringern. Darüberhinaus muss das Operationswerkzeug einfach und zuverlässig zu reinigen und zu sterilisieren sein.

Diese Aufgabe wird mit einem Operationswerkzeug bzw. durch einen Operationswerkzeugsatz gelöst, wie es bzw. er durch die Merkmale im jeweiligen unabhängigen Patentanspruch 1 bzw. 7 charakterisiert ist. Besonders vorteilhafte Ausführungsbeispiele bzw. Weiterbildungen ergeben sich aus den Merkmalen der abhängigen Patentansprüche.

Das erfindungsgemässe Operationswerkzeug für den Einsatz im Operationssaal umfasst ein Griffteil sowie ein mit diesem Griffteil fest verbundenes Werkzeugteil, wobei die Verbindung von Griffteil und Werkzeugteil als lösbare Verbindung ausgebildet ist.

Somit ist es möglich, mit ein- und demselben Griffteil eine Vielzahl von verschiedenen Werkzeugteilen zu verbinden, wodurch die Lagerhaltung wesentlich verkleinert wird, weil nur noch eine wesentlich geringere Anzahl von Griffteilen gelagert werden muss, da eine ganze Anzahl von einzelnen Werkzeugteilen mit ein- und demselben Griffteil verbunden werden können.

Insbesondere ist es auch möglich, je nach Einsatzgebiet ein bestimmtes Werkzeugteil mit einem bestimmten Typ von Griffteil zu kombinieren, wodurch die Vielfalt der Operationswerkzeuge erhalten oder sogar vergrössert wird. Nach dem Einsatz ist die feste Verbindung von Griffteil und Werkzeugteil wieder zu lösen und die Einzelteile sind zu reinigen und zu sterilisieren. Anschliessend stehen das Griffteil und das Werkzeugteil wieder - für einen Einsatz zur Verfügung, wobei dann eben mit dem gleichen Griffteil ein völlig unterschiedliches Werkzeugteil für ein völlig anderes Einsatzgebiet verbunden werden kann.

Ein weiterer Vorteil ist, dass bei einer sterilen Lagerung der Griffteile und der Werkzeugteile der operierende Arzt vor der Operation zunächst eine Anzahl von Werkzeugteilen und eine Anzahl von Griffteilen auswählen kann, die er möglicherweise bei der Operation benötigt, und die tatsächlich benötigten Operationswerkzeuge dann erst im Operationssaal endgültig zusammenstellen oder von assistierendem Personal zusammenstellen lassen kann.

Ein vorteilhaftes Ausführungsbeispiel des erfindungsgemässen Operationswerkzeugs ist mit einer Aufnahme am distalen Ende des Griffteils ausgestattet, in welche das Werkzeugteil eingebracht wird, wobei am Werkzeugteil und an der Aufnahme Mittel vorgesehen sind, welche die feste Verbindung von Griffteil und Werkzeugteil bewirken.

Diese Aufnahme für das Werkzeugteil umfasst vorzugsweise eine aus einem harten, verschleissarmen Material hergestellte Hülse, die unlösbar mit dem Griffteil verbunden ist. Die Hülse kann z.B. aus Keramik oder einem harten, verschleissarmen Kunststoff hergestellt sein. Vorzugsweise ist sie jedoch aus einem harten, verschleissarmen nicht-rostenden Metall wie z.B. aus einem Edelstahl hergestellt und kann in einen den Handgriff bildenden Kunststoff eingegossen oder eingespritzt worden sein, sodass das gesamte Griffteil zuverlässig reinigbar und sterilisierbar ist. Die separate Herstellbarkeit der Hülse und das erst an diese Herstellung sich anschliessende Eingiessen oder Einspritzen in einen Kunststoff bedeutet auch von der Herstellungsseite eine Vereinfachung.

Erfindungsgemäß ist die Verbindung von Griffteil und Werkzeugteil als Schraubverbindung ausgebildet. Dies ist einerseits eine sichere, andererseits eine einfach lösbare und auch zuverlässig herstellbare Verbindung.

Erfindungsgemäß weist das Griffteil bzw. die Hülse einen Anschlag für das proximale Ende des Werkzeugteils auf. Die Schraubverbindung umfasst eine Spann-Manschette mit einem Aussengewinde sowie ein mit diesem Aussengewinde der Spann-Manschette zusammenwirkendes Innengewinde im Griffteil bzw. in der Hülse. Die Spann-Manschette umschliesst das Werkzeugteil und ist in Richtung der Längsachse des Werkzeugteils am Werkzeugteil entlang bewegbar, bis Anschlagmittel, die am Werkzeugteil und an der Spann-Manschette vorgesehen sind, aneinander anstossen. Hierber kann zunächst das Werkzeugteil in das Griffteil bzw. in die Hülse eingebracht werden, bis das proximale Ende des Werkzeugteils gegen den im Griffteil bzw. in der Hülse vorgesehenen Anschlag stösst. Anschliessend kann das Aussengewinde der Spann-Manschette in das Innengewinde im Griffteil bzw. in der Hülse hinein eingeschraubt werden, bis die Anschlagmittel der Spann-Manschette gegen die Anschlagmittel am Werkzeugteil anstossen, sodass das Werkzeugteil dann gegen axiale Verschiebbarkeit gesichert ist.

Bei einer vorteilhaften Weiterbildung dieses Ausführungsbeispiels sind sowohl der Anschlag im Griffteil bzw. in der Hülse als auch das proximale Ende des Werkzeugteils kreiskegelförmig konisch ausgebildet. Ferner ist die Spann-Manschette an ihrem proximalen Ende mit dem Aussengewinde versehen und weist an ihrem distalen Ende einen nach innen abstehenden Vorsprung auf. Dieser Vorsprung ragt in eine Aussparung aussen auf dem Werkzeugteil hinein, sodass er die Bewegung der Spann-Manschette in Richtung der Längsachse des Werkzeugsteils begrenzt.

Die kreiskegelförmig konische Ausbildung des proximalen Endes des Werkzeugteils sowie des Anschlags im Griffteil bzw. in der Hülse bewirken eine Zentrierung des Werkzeugteils beim Einbringen des Werkzeugteils in das Griffteil. Nach dem Einbringen des Werkzeugteils wird das Aussengewinde der Spann-Manschette in das Innengewinde des Griffteils bzw. der Hülse hinein eingeschraubt. Dadurch wird der nach innen von der Spann-Manschette abstehende Vorsprung zusammen mit der Spann-Manschette in Richtung der Längsachse des Werkzeugteils entlang dem Werkzeugteil bewegt, und zwar in proximaler Richtung. Das Einschrauben - der Spann-Manschette erfolgt so lange, bis der nach innen von der Spann-Manschette abstehende Vorsprung ein weiteres Einschrauben der Spann-Manschette verhindert, weil er z.B. gegen eine Endfläche der Aussparung anstösst. Das Werkzeugteil ist dann gegen axiale Verschiebbarkeit gesichert und gleichzeitig zentriert.

Bei sämtlichen vorgenannten Ausführungsbeispielen weisen sowohl das Griffteil bzw. die Hülse als auch das Werkzeugteil jeweils einen Bereich auf, der nicht-rotationssymmetrisch ausgebildet ist. Diese nichtrotationssymetrisch ausgebildeten Bereiche sind von der Form und von ihren Abmessungen her zueinander passend ausgebildet, und der nicht-rotationssymmetrische Bereich des Griffteils bzw. der Hülse umschliesst den entsprechenden nicht-rotationssymmetrischen Bereich des Werkzeugteils, um ein Drehmoment übertragen zu können. Somit ist die axiale Sicherung des Werkzeugteils im Griffteil gegen Herausgleiten und die Übertragung von Drehmomenten funktional voneinander getrennt.

Bei einer Weiterbildung weist der nicht-rotationssymmetrische Bereich einen rechteckigen, vorzugsweise quadratischen Querschnitt auf. Ein solcher Querschnitt ist herstellungstechnisch einfach und gut geeignet für die Übertragung von Drehmomenten.

Der erfindungsgemässe Operationswerkzeugsatz umfasst wenigstens ein Griffteil sowie mehrere Werkzeugteile, wobei die Werkzeugteile in ihrem proximalen Bereich so ausgebildet sind, dass unterschiedliche Werkzeugteile von ein- und demselben Griffteil aufgenommen und fest mit diesem verbunden werden können. Dadurch wird die Werkzeugvielfalt erhalten oder sogar vergrössert und gleichzeitig der Lageraufwand verringert. Wie bereits oben erläutert, kann der Arzt vor der Operation sogar eine Anzahl von Griffteilen und eine Anzahl von Werkzeugteilen auswählen, die er möglicherweise bei der Operation benötigt, und erst im Operationssaal die gewünschte Kombination von Griffteil und Werkzeugteil zusammenstellen bzw. von assistierendem Personal zusammenstellen lassen.

Im folgenden wird die Erfindung anhand der Zeichnung näher erläutert. Dabei zeigen in schematischer Darstellung und/oder im Schnitt:
- Fig. 1: ein Ausführungsbeispiel eines erfindungsgemässen Operationswerkzeugs, bei welchem Griffteil und Werkzeugteil mit Hilfe einer Spann-Manschette fest, aber lösbar, miteinander -verbunden sind,
- Fig. 2: das Ausführungsbeispiel des Operationswerkzeugs aus Fig. 1, wobei jedoch Griffteil und Werkzeugteil noch nicht miteinander verbunden sind,

- Fig. 3: das Werkzeugteil des Operationswerkzeugs aus Fig. 1 ohne Spann-Manschette,
- Fig. 4: die Spann-Manschettte des Operationswerkzeugs aus Fig. 1, und
- Fig. 5: die Hülse des Griffteils des Operationswerkzeugs aus Fig. 1.

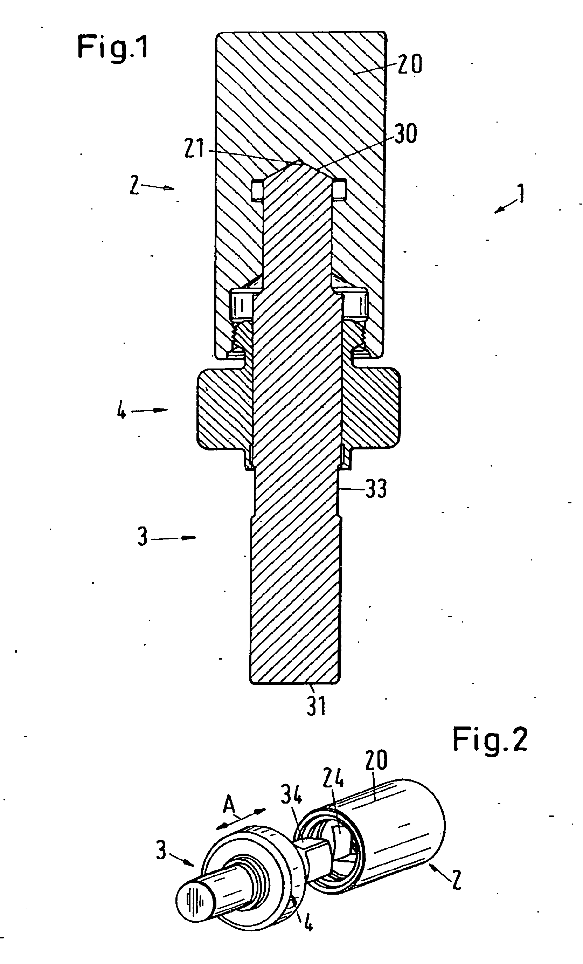

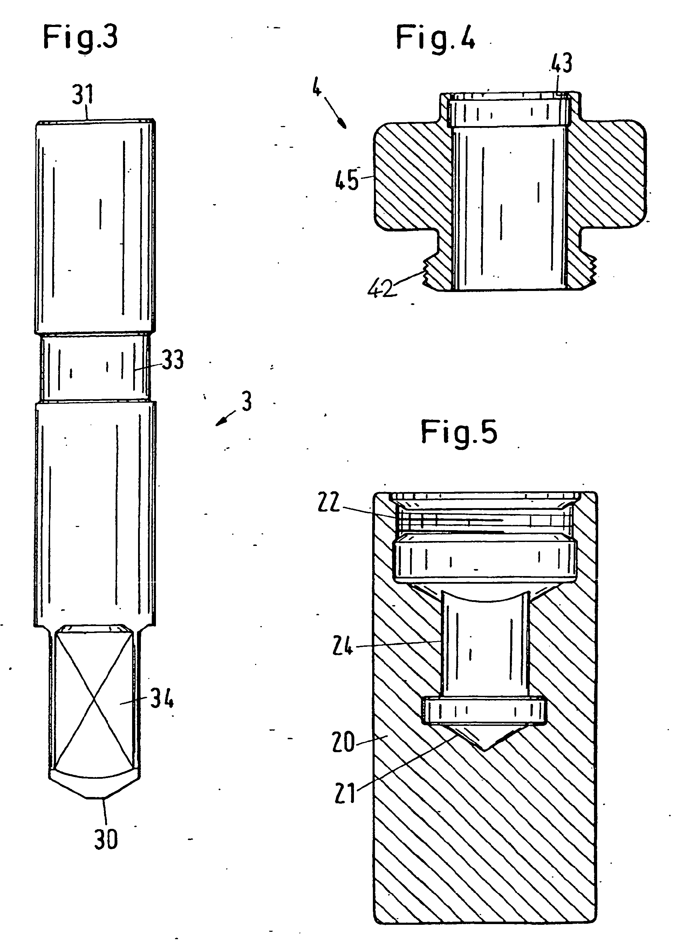

In Fig. 1 und Fig. 2 ist ein Ausführungsbeispiel eines erfindungsgemässen Operationswerkzeugs 1 dargestellt, wobei Fig. 1 eine Darstellung des Operationswerkzeugs im Zusammenbau in einer Darstellung im Längsschnitt zeigt, und Fig. 2 eine perspektivische Darstellung des Operationswerkzeugs 1, bei welchem Griffteil 2 und Werkzeugteil 3 nicht miteinander verbunden sind. Das Operationswerkzeug 1 umfasst das bereits angesprochene Griffteil 2 und das Werkzeugteil 3 sowie eine Spann-Manschette 4. Die einzelnen Teile werden anhand von Fig. 3, Fig. 4 und Fig. 5 noch genauer beschrieben.

Aus Fig. 1 erkennt man, dass die feste, aber lösbare Verbindung des Griffteils 2 und des Werkzeugteils 3 als Schraubverbindung ausgebildet ist. In Fig. 1 ist von dem Griffteil 2 nur eine aus einem harten, verschleissarmen Material, z.B. Edelstahl, hergestellte Hülse 20 zu erkennen (siehe auch Fig. 6), die eine Aufnahme für das proximale Ende 30 des Werkzeugteils 3 (siehe auch Fig. 4) darstellt. Diese Hülse 20 ist unlösbar mit dem Griffteil 2 verbunden, indem sie beispielsweise von einem Kunststoff umspritzt oder umgossen sein kann (nicht dargestellt). Aus zeichnerischen Gründen ist jedoch nur die Hülse 20 dargestellt, da für die Verbindung zwischen Werkzeugteil 3 und Griffteil 2 der die Hülse umgebende Kunststoff unerheblich ist.

Grundsätzlich sind die Hülse 20 des Griffteils 2, das Werkzeugteil 3, sowie die Spann-Manschette 4 separat herstellbare Teile, die anhand von Fig. 3, Fig. 4 und Fig. 5 noch beschrieben werden. Nach der Herstellung wird jedoch die Spann-Manschette 4 mit dem Werkzeugteil in noch genauer zu beschreibender Weise verbunden, sodass die Kombination aus Werkzeugteil 3 und Spann-Manschette 4 dann gemeinsam in die Hülse 20 des Griffteils 2 eingesetzt werden können bzw. nach einem erfolgten Einsatz des Operationswerkzeugs auch wieder gemeinsam aus der Hülse 20 des Griffteils 3 gelöst werden können. Dies ist durch die Pfeile A in Fig. 2 symbolisch angedeutet.

In Fig. 3 ist das Werkzeugteil 3 zu erkennen, wobei das distale Ende 31 des Werkzeugteils hier symbolisch nur als Stumpf einer Welle dargestellt ist. An diesem distalen Ende 31 des Werkzeugteils kann z.B. die Klinge eines Schraubendrehers angeformt sein oder eine Tastspitze oder ein Haken oder vieles andere mehr. Das proximale Ende 30 des Werkzeugteils 3 ist kreiskegelstumpfförmig konisch ausgebildet und wirkt mit einem entsprechenden kreiskegelförmig konisch ausgebildeten Anschlag 21 in der Hülse 20 zusammen. Dadurch wird das Werkzeugteil 3 beim Einbringen in die Hülse 20 und damit in das Griffteil 2 zentriert. Wie bereits zuvor erwähnt, wird jedoch die Spann-Manschette 4, die ja ein separat herstellbares Teil ist, vor dem Einbringen des Werkzeugteils 3 in das Griffteil 2 bzw. in die Hülse 20, mit dem Werkzeugteil verbunden.

Betrachtet man hierzu Fig. 4, in welcher die Spann-Manschette dargestellt ist, so erkennt man, dass die Spann-Manschette 4 an ihrem proximalen Ende ein Aussengewinde 42 aufweist, welches in ein entsprechendes Innengewinde 22 in der Hülse 20 einschraubbar ist. Zur "Befestigung" der Spann-Manschette 4 an dem Werkzeugteil 3 weist die Spann-Manschette 4 an ihrem distalen Ende einen umlaufenden Vorsprung 43 auf. Entsprechend ist an dem Werkzeugteil eine Aussparung 33 vorgesehen. Zur "Befestigung" der Spann-Manschette 4 am Werkzeugteil 3 wird die Spann-Manschette 4 über das Werkzeugteil 3 geschoben und der umlaufende Vorsprung 43 der Spann-Manschette 4 in die Aussparung 33 des Werkzeugteil 3 hineingebogen, z.B. gebördelt. Sodann ist die Spann-Manschette 4 im Rahmen der Begrenzung, die durch die Aussparung 33 dargestellt wird, bewegbar, denn der Vorsprung 43 schlägt nach dem Einbördeln am Ende der Aussparung 33 des Werkzeugteils 3 an, wenn er in Richtung der Längsachse entlang des Werkzeugteils 3 aussen auf dem Werkzeugteil bewegt wird. Das Werkzeugteil 3 mit auf dem Werkzeugteil 3 aufgebrachter Spann-Manschette 4 ist in Fig. 2 gut zu erkennen.

In Fig. 5 ist die Hülse 20 zu erkennen, welche die Aufnahme für das Werkzeugteil 3 darstellt. Man erkennt den kreiskegelförmigen Anschlag 21, gegen welchen das kreiskegelstumpfförmig ausgebildete proximale Ende des Werkzeugteils 3 beim Einbringen des Werkzeugteils 3 in die Hülse 20 hinein stösst. Ausserdem erkennt man das Innengewinde 22, in welches das Aussengewinde 42 der Spann-Manschette 4 beim Einschrauben der Spann-Manschette 4 in die Hülse 20 hinein eingreift. Schliesslich weist die Hülse 20 noch einen Bereich 24 auf, welcher als Vierkant ausgebildet ist und der beim Einbringen des Werkzeugteils.3 in die Hülse 20 hinein mit einem Bereich 34 des Werkzeugteils zusammenwirkt, der ebenfalls als Vierkant ausgebildet ist und bezüglich Form und Abmessungen passend zu dem Bereich 24 der Hülse 20 ausgebildet ist, um nach dem Einbringen des Werkzeugteils 3 in die Hülse 20 (und damit in das Griffteil) ein Drehmoment übertragen zu können. Grundsätzlich ist auch eine andere zueinander passende, nicht-rotationssymmetrische Ausgestaltung des Bereichs 24 der Hülse 20 und des Bereichs 34 des Werkzeugteils 3 möglich, um ein Drehmoment übertragen zu können. Der hier beschriebene Vierkant lässt sich jedoch auf einfache und zuverlässige Weise herstellen.

Die einzelnen Teile, die alle separat herstellbar sind, werden - wie zum Teil schon erläutert - in der folgenden Weise zu einem Operationswerkzeug verbunden. Zunächst wird die Spann-Manschette 4 über das Werkzeugteil 3 geschoben, bis der am distalen Ende nach innen abstehen Vorsprung 43 über der Aussparung 33 zu liegen kommt. Sodann wird der Vorsprung 43 in die Aussparung 33 hinein gebogen, z.B. gebördelt, sodass die Spann-Manschette 4 im Rahmen der axialen Ausdehnung der Aussparung 33 in Richtung der Längsachse des Werkzeugteils 3 aussen auf dem Werkzeugteil entlang bewegt werden kann. Das mit der Spann-Manschette 4 versehene Werkzeugteil 3 wird dann mit seinem proximalen Ende voran in die Hülse 20 eingebracht, und zwar derart, dass der Bereich 34 (Vierkant) des Werkzeugteils 3 in den Bereich 24 der Hülse 20 hinein passt. Beim Einbringen des Werkzeugteils 3 erfolgt zunächst am proximalen Ende des Werkzeugteils 3 eine Zentrierung, weil das kreiskegelstumpfförmig ausgebildete proximale Ende 30 des Werkzeugteils 3 gegen den kreiskegelförmig ausgebildeten Anschlag 21 der Hülse 20 zu liegen kommt.

Sodann wird das Aussengewinde 42 der Spann-Manschette 4 in das Innengewinde 22 der Hülse eingeschraubt, wodurch einerseits auch eine Zentrierung des Werkzeugteils am distalen Ende der Hülse 20 erfolgt und ausserdem die Spann-Manschette 4 aufgrund der Steigung des Gewindes in proximaler Richtung entlang der Längsachse des Werkzeugteils 3 bewegt wird. Der Bereich 45 der Spann-Manschette 4 kann z.B. mit einer Rändelung versehen sein, um das Einschrauben der Spann-Manschette 4 in die Hülse 20 hinein zu erleichtern. Die Spann-Manschette 4 wird so lange weiter in die Hülse 20 eingeschraubt, bis der nach innen abstehende Vorsprung 43 gegen die proximale Begrenzung der Aussparung 33 des Werkzeugteils 3 stösst. Das Werkzeugteil 3 ist so gegen axiales Herausgleiten gesichert (verspannt) und im übrigen - wie bereits erläutert auch zentriert. Über den Vierkant können Drehmomente übertragen werden.

## Patentansprüche

1. Operationswerkzeug (1) für den Einsatz im Operationssaal, welches ein Griffteil (2) umfasst sowie ein mit diesem Griffteil (2) fest verbundenes Werkzeugteil (3), wobei die Verbindung von Griffteil (2) und Werkzeugteil (3) als lösbare Schraubverbindung (22,42) ausgebildet ist,
wobei das Griffteil (2) einen Anschlag (21) für das proximale Ende (30) des Werkzeugteils (3) aufweist und die Schraubverbindung eine Spann-Manschette (4) mit einem Aussengewinde (42) sowie ein mit diesem Aussengewinde (42) der Spann-Manschette (4) zusammenwirkendes Innengewinde (22) im Griffteil (2) umfasst, wobei die Spann-Manschette (4) das Werkzeugteil (3) umschliesst und in Richtung der Längsachse des Werkzeugteils (3) am Werkzeugteil entlang bewegbar ist, bis Anschlagmittel, die am Werkzeugteil (3) und an der Spann-Manschette (4) vorgesehen sind, aneinander anstossen.

2. Operationswerkzeug nach Anspruch 1, mit einer Aufnahme am distalen Ende des Griffteils (2), in welche das Werkzeugteil (3) eingebracht wird, wobei am Werkzeugteil und an der Aufnahme Mittel (22,42) vorgesehen sind, welche die feste Verbindung von Griffteil (2) und Werkzeugteil (3) bewirken.

3. Operationswerkzeug nach Anspruch 2, bei welchem die Aufnahme für das Werkzeugteil eine aus einem harten, verschleissarmen Material hergestellte Hülse (20) umfasst, die unlösbar mit dem Griffteil (2) verbunden ist.

4. Operationswerkzeug nach einem der vorhergehenden Ansprüche, **dadurch gekennzeichnet, dass** sowohl der Anschlag (21) im Griffteil (2) als auch das proximale Ende (30) des Werkzeugteils (3) kreiskegelförmig konisch ausgebildet sind, und dass ferner die Spann-Manschette (4) an ihrem proximalen Ende mit dem Aussengewinde (42) versehen ist und an ihrem distalen Ende einen nach innen abstehenden Vorsprung (43) aufweist, der in eine Aussparung (33) aussen auf dem Werkzeugteil (3) hineinragt, sodass er die Bewegung der Spann-Manschette (4) in Richtung der Längsachse des Werkzeugteils (3) begrenzt.

5. Operationswerkzeug nach einem der vorangehenden Ansprüche, **dadurch gekennzeichnet, dass** sowohl das Griffteil (2) als auch das Werkzeugteil (3) jeweils einen Bereich (24,34) aufweisen, der nicht-rotationssymmetrisch ausgebildet ist, wobei diese nichtrotationssymetrisch ausgebildeten Bereiche (24,34) von der Form und von ihren Abmessungen her zueinander passend ausgebildet sind und der nicht-rotationssymmetrische Bereich (24) des Griffteils (2) den entsprechenden nicht-rotationssymetrischen Bereich (34) des Werkzeugteils (3) umschliesst, um ein Drehmoment übertragen zu können.

6. Operationswerkzeug nach Anspruch 5, **dadurch gekennzeichnet, dass** der nicht-rotationssymmetrische Bereich (24,34) einen rechteckigen, vorzugsweise quadratischen Querschnitt aufweist.

7. Operationswerkzeugsatz, umfassend wenigstens ein Griffteil (2) sowie mehrere Werkzeugteile (3), wobei die Werkzeugteile (3) in ihrem proximalen Bereich so ausgebildet sind, dass unterschiedliche Werkzeugteile (3) von ein- und demselben Griffteil (2) aufgenommen und durch eine lösbare Schraubverbindung fest mit diesem verbunden werden können,
wobei das Griffteil (2)
einen Anschlag (21) für das proximale Ende (30) des jeweiligen Werkzeugteils (3) aufweist und die Schraubverbindung eine Spann-Manschette (4) mit einem Aussengewinde (42) sowie ein mit diesem Aussengewinde (42) der Spann-Manschette (4) zusammenwirkendes Innengewinde (22) im Griffteil (2) umfasst, wobei die Spann-Manschette (4) das Werkzeugteil (3) umschliesst und in Richtung der Längsachse des Werkzeugteils (3) am Werkzeugteil entlang bewegbar ist, bis Anschlagmittel, die am Werkzeugteil (3) und an der Spann-Manschette (4) vorgesehen sind, aneinander anstossen.

## Claims

1. Operating tool (1) for use in the operating room, comprising a grip part (2) and a tool part (3) which is firmly connected to this grip part (2), with the connection of the grip part (2) and the tool part (3) being formed as a releasable screw connection (22, 42), wherein the grip part (2) has an abutment (21) for the proximal end (30) of the tool part (3) and the screw connection comprises a clamping collar (4) with an external thread (42) and an internal thread (22) in the grip part (2) which cooperates with this external thread (42) of the clamping collar (4), with the clamping collar (4) surrounding the tool part (3) and being movable along the tool part in the direction of the longitudinal axis of the tool part (3) until abutment means which are provided at the tool part (3) and at the clamping collar (4) abut one another.

2. Operating tool in accordance with claim 1, comprising a reception at the distal end of the grip part (2) into which the tool part (3) is introduced, with means (22, 42) being provided at the tool part and at the reception which effect the firm connection of the grip part (2) and the tool part (3).

3. Operating tool in accordance with claim 2, in which the reception for the tool part comprises a sleeve (20) which is manufactured of a hard, low-wear material and which is non-releasably connected to the grip part (2).

4. Operating tool in accordance with any one of the preceding claims, **characterized in that** both the abutment (21) in the grip part or in the sleeve (20) respectively and the proximal end (30) of the tool part (3) are formed to be circularly conical, and **in that** furthermore the clamping collar (4) is provided with the external thread (42) at its proximal end and has at its distal end an inwardly projecting protrusion (43) which projects into a cut-out (33) on the outside of the tool part (3) so that it bounds the movement of the clamping collar (4) in the direction of the longitudinal axis of the tool part (3).

5. Operating tool in accordance with any one of the preceding claims, **characterized in that** both the grip part (2) and the tool part (3) in each case have a region (24, 34) which is formed non-rotationally symmetrically, with these non-rotationally symmetrical regions (24, 34) , being designed to fit together with one another in regard to their shapes and their dimensions and with the non rotationally symmetrical region (24) of the grip part (2) surrounding the corresponding non rotationally symmetrical region (34) of the tool part (3) in order to be able to transmit a torque.

6. Operating tool in accordance with claim 5, **characterized in that** the non-rotationally symmetrical region (24, 34) has a rectangular cross-section, preferably a square cross-section.

7. Operating tool set comprising at least one grip part (2) and a plurality of tool parts (3), with the tool parts (3) being designed in their proximal region such that different tool parts (3) can be received by one and the same grip part (2) and firmly connected to the latter by a releasable screw connection, wherein the grip part (2) has an abutment (21) for the proximal end (30) of the tool part (3) and the screw connection comprises a clamping collar (4) with an external thread (42) and an internal thread (22) in the grip part (2) which cooperates with this external thread (42) of the clamping collar (4), with the clamping collar (4) surrounding the tool part (3) and being movable along the tool part in the direction of the longitudinal axis of the tool part (3) until abutment means which are provided at the tool part (3) and at the clamping collar (4) abut one another.

## Revendications

1. Outil d'opération (1) destiné à être utilisé en salle d'opérations, lequel comprend une partie de préhension (2) ainsi qu'une partie d'outil (3) reliée solidaire à cette partie de préhension (2), la liaison entre la partie de préhension (2) et la partie d'outil (3) étant réalisée sous forme de liaison par vissage (22, 42) détachable, dans lequel la partie de préhension (2) présente une butée (21) pour l'extrémité proximale (30) de la partie d'outil (3) et la liaison par vissage comprend une manchette de serrage (4) avec un pas de vis extérieur (42) ainsi qu'un pas de vis intérieur (22) dans la partie de préhension (2) coopérant avec ce pas de vis extérieur (42) de la manchette de serrage (4), la manchette de serrage (4) entourant la partie d'outil (3) et étant déplaçable le long de la partie d'outil en direction de l'axe longitudinal de la partie d'outil (3) jusqu'à ce que des moyens de butée qui sont prévus sur la partie d'outil (3) et sur la manchette de serrage (4) butent l'un contre l'autre.

2. Outil d'opération selon la revendication 1, comprenant un logement à l'extrémité distale de la partie de préhension (2) dans lequel est introduite la partie d'outil (3), et sur la partie d'outil et sur le logement sont prévus des moyens (22, 24) qui réalisent la liaison solidaire entre la partie de préhension (2) et la partie d'outil (3).

3. Outil d'opération selon la revendication 2, dans lequel le logement pour la partie d'outil comprend une douille (20) réalisée en un matériau dur peu sujet à l'usure qui est relié de manière indétachable à la partie de préhension (2).

4. Outil d'opération selon l'une des revendications précédentes, **caractérisé en ce que** tant la butée (21) dans la partie de préhension (2) que l'extrémité proximale (30) de la partie d'outil (3) sont réalisées en forme de cône de révolution et **en ce qu'**en outre, la manchette de serrage (4) est pourvue du pas de vis extérieur (42) à son extrémité proximale et présente à son extrémité distale une saillie (43) dépassant vers l'intérieur qui plonge dans un évidement (33) à l'extérieur sur la partie d'outil (3), de telle sorte qu'elle limite le mouvement de la manchette de serrage (4) en direction de l'axe longitudinal de la partie d'outil (3).

5. Outil d'opération selon l'une des revendications précédentes, **caractérisé en ce que** tant la partie de préhension (2) que la partie d'outil (3) présentent chacune une région (24, 34) qui n'est pas réalisée à symétrie de révolution, ces régions réalisées sans symétrie de révolution (24, 34) étant réalisées en étant adaptées l'une à l'autre du point de la forme et du point de leurs dimensions et la région réalisée sans symétrie de révolution (24) de la partie de préhension (2) entourant la partie sans symétrie de révolution (34) correspondante de la partie d'outil (3) pour pouvoir transmettre un couple de rotation.

6. Outil d'opération selon la revendication 5, **caractérisé en ce que** la région sans symétrie de révolution (24, 34) présente une section transversale rectangulaire, de préférence carrée.

7. Jeu d'outils d'opération comprenant au moins une partie de préhension (2) ainsi que plusieurs parties d'outil (3), les parties d'outil (3) étant réalisées dans leur région proximale de telle sorte que différentes parties d'outil (3) peuvent être reçues par une seule et même partie de préhension (2) et être fixées de manière solidaire à celle-ci par une liaison par vissage détachable, la partie de préhension (2) présentant une butée (21) pour l'extrémité proximale (30) de la partie d'outil (3) respective, et la liaison par vissage comprend une manchette de serrage (4) avec un pas de vis extérieur (42) ainsi qu'un pas de vis intérieur (22) dans la partie de préhension (2) coopérant avec ce pas de vis extérieur (42) de la manchette de serrage (4), la manchette de serrage (4) entourant la partie d'outil (3) et étant déplaçable le long de la partie d'outil (3) en direction de l'axe longitudinal de la partie d'outil (3) jusqu'à ce que des moyens de butée qui sont prévus sur la partie d'outil (3) et sur la manchette de serrage (4) butent l'un contre l'autre.
